# EUROPEAN PATENT APPLICATION

(11) **EP 4 785 897 A1**
(43) Date of publication of application: **05.08.2026**
(21) Application number: 24876468.0
(22) Date of filing: 30.09.2024
(51) Int. Cl.: A61B 34/35, A61B 34/30

(54) **INSTRUMENT-HOLDING ARM MECHANISM, MULTI-AXIS MOVEMENT DEVICE, AND SURGICAL ROBOT**

(30) Priority: 09.10.2023 CN 202311306760
(71) Applicant: Cornerstone Technology (Shenzhen) Limited, Shenzhen, Guandong 518066 (CN)
(72) Inventor: CAI, Changrong, Shenzhen, Guangdong 518066 (CN); TI, Xiaocong, Shenzhen, Guangdong 518066 (CN)
(74) Representative: Balder IP Law, S.L.
(86) International application number: PCT/CN2024/122904
(87) International publication number: WO 2025/077653

(57) **Abstract**

An instrument holder (13), a multi-axis motion device (10), and a surgical robot (1) are provided. The instrument holder (13) includes an arm body (100), an actuator (200), a lead screw (300), a first connecting component (400), and a second connecting component (500). The arm body (100) defines a first cavity (110) extending between two ends thereof in a first direction, and further defines a through slot (120) extending in the first direction and communicating the first cavity (110) with an exterior of the arm body (100). The actuator (200) is fixed to one end of the arm body (100), and the lead screw (300) is connected to the actuator (200) and is driven to rotate by means of the actuator (200). The first connecting component (400) is located in the first cavity (110), and can move in a reciprocating manner in an axis direction of the lead screw (300) under the driving of the lead screw (300). The second connecting component (500) is configured to mount an instrument driver (12), and is fixedly connected to the first connecting component (400).

## Description

### CROSS-REFERENCE TO RELATED APPLICATIONS

The present disclosure claims the benefits of and priority to Chinese Patent Application No. 202311306760.0, filed on October 09, 2023, the entire disclosure of which is hereby incorporated by reference, in its entirety, for all that it teaches and for all purposes.

### TECHNICAL FIELD

The present disclosure relates to the technical field of medical equipment, and in particular to an instrument holder, a multi-axis motion device, and a surgical robot.

### BACKGROUND

Currently, surgical robots are widely used in various surgical procedures by virtue of their advantages such as precise positioning, stable operation, high flexibility, extensive working range, and immunity to radiation and infection.

In related art, a surgical robot generally includes a multi-axis motion device, which includes a plurality of manipulators, each having a distal end capable of multi-degree-of-freedom motion. An instrument holder is mounted at the distal end of the manipulator to drive an instrument driver to move in a predetermined direction. However, in the related art, during assembly of the instrument holder, the use of numerous components and an unreasonable assembly sequence can lead to error accumulation, resulting in significant assembly errors and making calibration difficult.

### SUMMARY

The present disclosure provides an instrument holder, a multi-axis motion device, and a surgical robot. The instrument holder features minimal assembly errors and easy calibration, thereby improving the motion precision of the multi-axis motion device and the overall surgical accuracy of the surgical robot.

To realize the above objective, the present disclosure provides an instrument holder, including: an arm body, an actuator fixed to an end of the arm body, a lead screw, a first connecting component, and a second connecting component. The arm body defines a first cavity extending between two ends of the arm body in a first direction, the arm body further defines a through slot for communicating the first cavity with an exterior of the arm body, the through slot extends in the first direction and includes a first end and a second end oppositely disposed in the first direction, and the arm body includes a first mating surface disposed within the first cavity. At least a portion of the lead screw extends within the first cavity, the lead screw is coupled to the actuator and configured to be driven by the actuator to rotate about an axis parallel to the first direction. The first connecting component is located within the first cavity. The first connecting component is threadedly engaged with the lead screw and configured to be driven by the lead screw to reciprocate in an axial direction of the lead screw. A surface of the first connecting component defines a second mating surface. The second connecting component is configured for mounting an instrument driver. The second connecting component is partly located outside the arm body, and passes through the through slot to be fixed to the first connecting component. The first connecting component and the second connecting component are reciprocatable between the first end and the second end of the through slot. The first mating surface mates with the second mating surface to prevent the first connecting component from rotating about the axis of the lead screw. The second connecting component is free of contact with the arm body.

According to the present disclosure, during assembly of the instrument holder, the actuator is mounted and fixed to an end of the arm body, while the lead screw and the first connecting component are pre-assembled and then mounted to the arm body together. In this process, the lead screw and the first connecting component are inserted into the first cavity from the other end of the arm body, such that the first mating surface engages with the second mating surface, preventing the first connecting component from rotating about the axis of the lead screw and allowing the lead screw to drive the first connecting component to reciprocate in the axial direction of the lead screw. After the installation of the first connecting component is completed, the mounting position of the lead screw is adjusted such that the axis of the lead screw is parallel to the first direction, and the lead screw is coupled to the actuator. Finally, a portion of the second connecting component passes through the through slot and is fixed to the first connecting component, such that a portion of the second connecting component is disposed outside the arm body, facilitating assembly with the instrument driver. Thus, by providing the first connecting component, the instrument holder achieves both a sliding connection with the arm body and a threaded engagement with the lead screw, thereby reducing the number of assembly components and consequently reducing assembly errors. Additionally, assembling the lead screw and the first connecting component together before mounting them to the arm body effectively minimizes error accumulation, resulting in a small parallelism error between the axis of the lead screw and the first direction, facilitating calibration. Further, after installation, the second connecting component is free of contact with the arm body, thus avoiding any impact on the transmission precision among the first connecting component, the lead screw, and the arm body, which contributes to improving the transmission precision of the instrument holder.

According to a second aspect, the present disclosure provides a multi-axis motion device, including a manipulator, an instrument driver, and the above instrument holder. The instrument holder is mounted at a distal end of the manipulator, and the instrument driver is coupled to the second connecting component.

The multi-axis motion device includes the above-described instrument holder, which provides high transmission precision and enables the instrument driver to move smoothly in the first direction, thereby ensuring high motion precision and excellent operational stability of the multi-axis motion device.

According to a third aspect, the present disclosure provides a surgical robot, including the above multi-axis motion device.

The surgical robot includes the above-described multi-axis motion device, which features high motion precision and excellent operational stability, thereby enhancing surgical accuracy of the surgical robot.

### DESCRIPTION OF DRAWINGS

To explain the technical solutions of the embodiments of the present disclosure more clearly, the following briefly introduces the accompanying drawings required for describing the embodiments of the present disclosure. For those of ordinary skill in the art, other drawings can be derived from these drawings without inventive effort.
FIG. 1 is a structural diagram of a surgical robot according to an embodiment of the present disclosure.
FIG. 2 is a structural diagram of a multi-axis motion device shown in FIG. 1.
FIG. 3 is an exploded view of the instrument holder shown in FIG. 2.
FIG. 4 is an assembled diagram of the instrument holder shown in FIG. 3.
FIG. 5 shows part of an instrument holder according to another embodiment of the present disclosure.
FIG. 6 is a semi-sectional view of the instrument holder shown in FIG. 4, taken along a third direction.
FIG. 7 is a semi-sectional view of the instrument holder shown in FIG. 5, taken along a third direction.
FIG. 8 is a part of a sectional view of the instrument holder shown in FIG. 4, taken along a first direction.
FIG. 9 is a diagram illustrating movement of the cable carrier of the instrument holder shown in FIG. 4 within the cable routing area.
FIG. 10 is an exploded view of an instrument holder according to another embodiment of the present disclosure.

### DESCRIPTION OF DRAWINGS

To make the objectives, technical solutions, and advantages of the embodiments of the present disclosure clearer, the embodiments of the present disclosure will be described in detail below with reference to the accompanying drawings. However, those of ordinary skill in the art will appreciate that, in the embodiments of the present disclosure, numerous technical details are set forth in order to provide a better understanding of the present disclosure. Nevertheless, the technical solutions sought to be protected by the present disclosure may be implemented even without these technical details and based on various changes and modifications of the following embodiments.

In the embodiments of the present disclosure, terms such as "upper," "lower," "left," "right," "front," "rear," "top," "bottom," "inner," "outer," "middle," "vertical," "horizontal," "lateral," and "longitudinal" and other indicating orientations or positional relationships are based on the orientations or positional relationships shown in the accompanying drawings. These terms are primarily used to better describe the present disclosure and its embodiments, and are not intended to require that the indicated device, element, or component have a specific orientation or be constructed and operated in a specific orientation.

In addition to indicating orientations or positional relationships, some of the above terms may also be used to indicate other meanings. For example, the term "upper" may in some cases also be used to indicate a certain attachment or connection relationship. Those of ordinary skill in the art can understand the specific meanings of these terms in the present disclosure based on the specific context.

Moreover, the technical terms "mounted," "arranged," "provided with," "defined," "coupled," and "coupled" should be broadly interpreted. For example, such terms may refer to a fixed connection, a detachable connection, or an integral structure; may refer to a mechanical connection or an electrical connection; may refer to a direct connection or an indirect connection via an intermediate medium, or may refer to internal communication between two devices, elements, or components. Those of ordinary skill in the art will understand the specific meanings of the foregoing terms in the present disclosure based on the specific context.

It should be noted that, in the description of the embodiments of the present disclosure, relational terms such as first and second merely used to distinguish one entity or operation from another entity or operation, and should not be construed as necessarily require or imply any such actual relationship or order between these entities or operations. Moreover, the terms "include," "have," or any other variations thereof are intended to cover a non-exclusive inclusion, such that a process, method, article, or apparatus that comprises a list of elements includes not only those elements but also other elements not expressly listed, or elements inherent to such process, method, article, or apparatus. In the absence of further restrictions, an element defined by the phrase "including..." does not preclude the presence of additional identical elements in the process, method, article, or apparatus that includes the element.

It should be noted that the term "spaced apart" as used herein shall be understood in a broad sense, including but not limited to being spaced apart, arranged at a distance, and the like.

When an element is described as being perpendicular or approximately perpendicular to another element, it means that the two elements are nominally perpendicular to each other, although a certain perpendicularity error may exist due to manufacturing and assembly tolerances. The terms "perpendicular," "horizontal," "left," "right," and similar expressions used herein are for illustrative purposes only and are not intended to limit the embodiments to the specific orientations described.

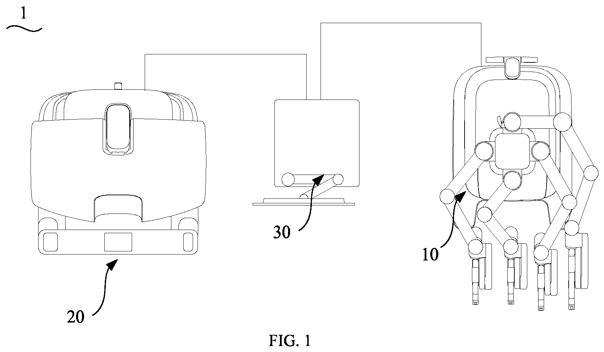

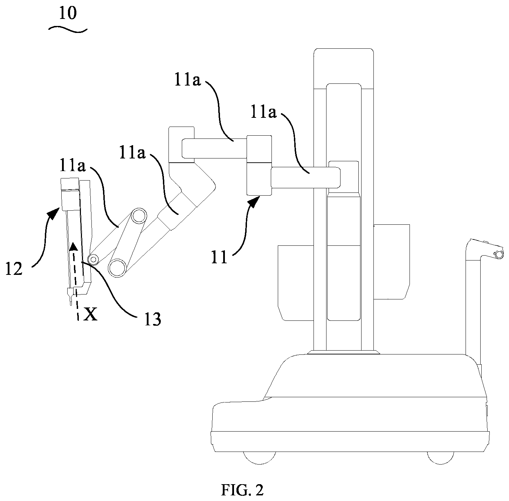

As shown in FIG. 1 and FIG. 2, the present disclosure provides a surgical robot 1, including a multi-axis motion device 10 configured to drive a surgical instrument to move, so as to perform corresponding surgical actions. The surgical robot 1 further includes a control device 20 and an imaging device 30. The control device 20 is communicatively coupled to both the imaging device 30 and the multi-axis motion device 10, allowing a surgeon to control the multi-axis motion device 10 via the control device 20 to perform surgical instrument operations, during which the surgeon can use the imaging device 30 for surgical observation.

In some embodiments, the control device 20 includes a display mechanism for showing an environment of the surgical instrument and a control mechanism for a surgeon to operate. The display mechanism is provided with an observation window, which is convenient for the surgeon to observe. The surgeon operates the control mechanism to move the multi-axis motion device 10, thereby performing the surgical instrument actions.

In some embodiments, the control device 20 further includes control switches that can be easily touched or pressed by hand or foot, enabling various operations and human-machine interaction.

In some embodiments, the imaging device 30 includes at least one selected from a group consisting of a display screen, an endoscope controller, a system electronic device, and an image processor.

As shown in FIG. 2, in some embodiments, the multi-axis motion device 10 includes a manipulator 11 and an instrument driver 12. The manipulator 11 includes at least two sequentially coupled links 11a, with adjacent links 1la capable of moving relative to each other with predetermined degrees of freedom, thereby allowing a distal end of the manipulator 11 to achieve multi-degree-of-freedom motion. The instrument driver 12 is mounted at the distal end of the manipulator 11 to drive the surgical instrument to perform operations including gripping, yawing, and pitching.

As shown in FIG. 2, in some embodiments, the multi-axis motion device 10 further includes an instrument holder 13 mounted at the distal end of the manipulator 11. The instrument holder 13 is coupled to the instrument driver 12 to drive the instrument driver 12 to move in a first direction (i.e., the X-axis direction shown in FIG. 2). In this way, the operator can adjust the position of the instrument driver 12 relative to the manipulator 11 via the instrument holder 13, thereby facilitating surgical operations and enabling multi-degree-of-freedom motion.

The movement accuracy and smoothness of the instrument driver 12 in the first direction affect both the motion precision of the multi-axis motion device 10 and the surgical operation precision of the surgical robot 1.

During the assembly process of a conventional instrument holder, the large number of components makes it prone to error accumulation, resulting in a large assembly tolerances and increased calibration difficulty. For example, when the conventional instrument holder is assembled, the slider and the lead screw are typically installed independently on the arm body before being calibrated, making it difficult to ensure parallelism between the axial direction of the lead screw and the sliding direction of the slider. This making it challenging to improve the movement accuracy of the instrument holder in driving the instrument driver to move in a predetermined direction. Even worse, due to significant parallelism error between the slider and the lead screw, the lead screw is prone to jamming, which affects the smoothness of movement of the instrument driver in the predetermined direction.

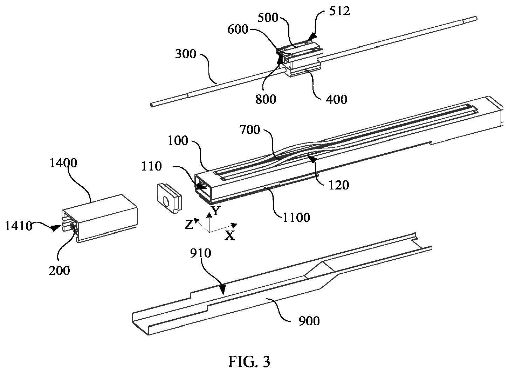

Based on this, in some embodiments of the present disclosure, as shown in FIG. 3 to FIG. 6, the instrument holder 13 includes an arm body 100, an actuator 200, a lead screw 300, a first connecting component 400, and a second connecting component 500 configured to mount the instrument driver 12. The arm body 100 serves as a load-bearing structure. The arm body 100 defines a first cavity 110 extending between both ends of the arm body 100 in the first direction. The arm body 100 further defines a through slot 120, allowing for the first cavity 110 communicating with the exterior of the arm body 100. The through slot 120 extends in the first direction, and has a first end and a second end oppositely disposed in the first direction. The arm body 100 includes a first mating surface 130 provided within the first cavity 110. The first cavity 110 provides protection for the lead screw 300 and the first connecting component 400, preventing solid contaminants such as dust from polluting the lead screw 300 and the first connecting component 400, which could otherwise reduce the service life of the lead screw 300 and the first connecting component 400. The arrangement of the through slot 120 does not affect the connection between the second connecting component 500 and the first connecting component 400, thereby facilitating mounting of the instrument driver 12 onto the second connecting component 500.

The actuator 200 is fixed to an end of the arm body 100. At least a portion of the lead screw 300 extends within the first cavity 110. The lead screw 300 is coupled to the actuator 200 and is driven by the actuator 200 to rotate. The lead screw 300 rotates about an axis parallel to the first direction. The first connecting component 400 is located within the first cavity 110. The first connecting component 400 is in threaded engagement with the lead screw 300 (the threaded engagement may be a direct threaded engagement or a ball screw engagement), and is reciprocatable in the axial direction of the lead screw 300 when driven by the lead screw 300. A surface of the first connecting component 400 defines a second mating surface 410. The second connecting component 500 is configured to mount the instrument driver 12. The second connecting component 500 is partly located outside the arm body 100, and passes through the through slot 120 to be fixed to the first connecting component 400. The first connecting component 400 and the second connecting component 500 are reciprocatable between the first end and the second end of the through slot 120. The first mating surface 130 cooperates with the second mating surface 410 to prevent the first connecting component 400 from rotating about the axis of the lead screw 300. During assembly of the instrument holder 13, the actuator 200 is first fixed to one end of the arm body 100. The lead screw 300 and the first connecting component 400 are pre-assembled and then inserted into the arm body 100 from the opposite end of the arm body 100. In this process, the lead screw 300 and the first connecting component 400 are placed into the first cavity 110, such that the first mating surface 130 engages with the second mating surface 410 to prevent rotation of the first connecting component 400 about the axis of the lead screw 300, thereby enabling the lead screw 300 to drive the first connecting component 400 to reciprocate in the axial direction of the lead screw 300. After the installation of the first connecting component 400 is completed, the installation position of the lead screw 300 is adjusted to ensure that the axis of the lead screw 300 is parallel to the first direction, and such that the lead screw 300 is coupled to the actuator 200. Finally, a portion of the second connecting component 500 is inserted through the through slot 120 and fixed to the first connecting component 400, with the remaining portion of the second connecting component 500 located outside the arm body 100 for subsequent assembly with the instrument driver 12.

In other words, the instrument holder 13 achieves sliding connection with the arm body 100 and threaded transmission cooperation with the lead screw 300 by providing the first connecting component 400, so as to reduce the number of assembly components and further minimize assembly errors. Moreover, by assembling the lead screw 300 and the first connecting component 400 first and then assembling them together onto the arm body 100, error accumulation is effectively reduced, resulting in a small parallelism error between the axis of the lead screw 300 and the first direction, facilitating calibration. At the same time, after the second connecting component 500 is installed, the second connecting component 500 is free of contact with the arm body 100, thus not affecting the transmission precision between the first connecting component 400 and the lead screw 300 with respect to the arm body 100, thereby improving the transmission precision of the instrument holder 13.

It should be understood that the actuator 200 is mounted to the arm body 100 from one end of the arm body 100, and the first connecting component 400 and the lead screw 300 are mounted to the arm body 100 from the other end of the arm body 100, facilitating assembly. Moreover, when adjusting the assembly position of the first connecting component 400 relative to the arm body 100, it is easy to simultaneously calibrate the positional relationship among the lead screw 300, the first connecting component 400, and the arm body 100, making calibration convenient. This prevents the lead screw 300 from jamming during rotation and ensures a small parallelism error between the axis of the lead screw 300 and the first direction.

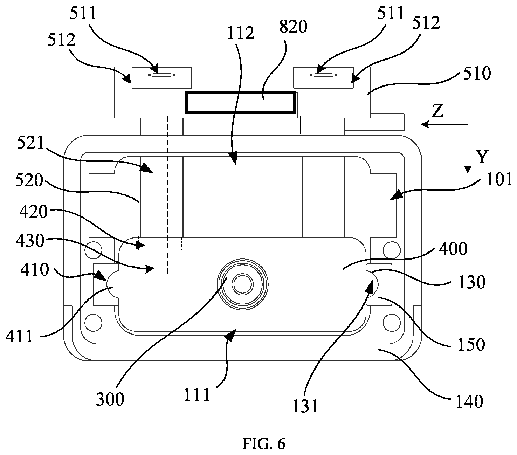

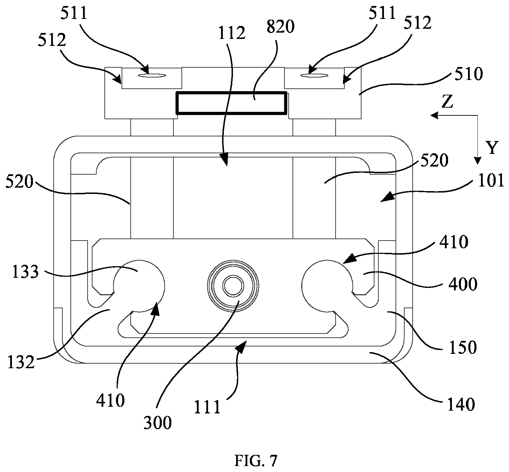

The second connecting component 500 can be implemented in various configurations. For example, as shown in FIG. 6 or FIG. 7, in some embodiments, the second connecting component 500 includes a first connecting body 510 and a second connecting body 520. The first connecting body 510 is configured to mount the instrument driver 12, and at least a portion of the first connecting body 510 is located outside the arm body 100. The second connecting body 520 includes an end located within the first cavity 110 and fixed to the first connecting component 400, and another end fixed to the first connecting body 510. Therefore, by way of the cooperation between the first connecting body 510 and the second connecting body 520, it is convenient to mount the instrument driver 12 via the first connecting body 510, while the fixed connection between the second connecting body 520 and the first connecting component 400 enables the first connecting component 400 to drive the instrument driver 12 to move in the first direction.

In some embodiments, the first connecting body 510 and the second connecting body 520 are configured as an integral structure. The first connecting body 510 and the second connecting body 520 are integrally formed to obtain the second connecting component 500, which reduces assembly steps and improves the assembling efficiency of the instrument holder 13.

In some other embodiments, the first connecting body 510 and the second connecting body 520 are configured as separate structures. The first connecting body 510 and the second connecting body 520 are manufactured separately and then assembled to form the second connecting component 500, thereby reducing the manufacturing difficulty of the second connecting component 500.

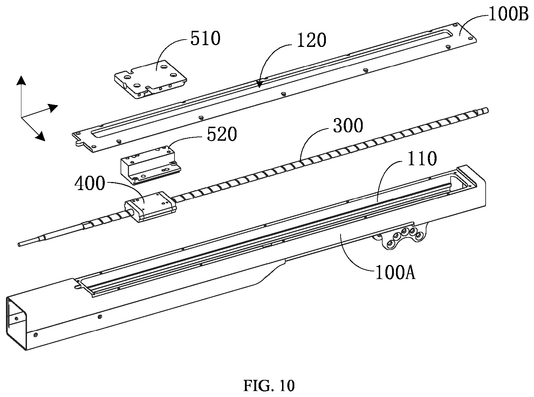

FIG. 10 shows the separate structures of the first connecting body 510 and the second connecting body 520 according to an embodiment of the present disclosure. As show in FIG. 10, the second connecting body 520 is configured in a non-standard shape, for example, an L-shaped structure or an inverted T-shaped structure. During assembly, the second connecting body 520 is first fastened to the first connecting component 400 by screws, and then the first connecting body 510 is fixed to the second connecting body 520.

In some embodiments, as shown in FIG. 10, the second connecting body 520 includes a first body (not labeled) coupled to the first connecting component 400 and a second body (not labeled) coupled to the first connecting body 510. At least a portion of the second body protrudes from the first body.

As shown in FIG. 6 or FIG. 7, in some embodiments, the second connecting body 520 passes through the through slot 120. The other end of the second connecting body 520 is located outside the arm body 100 and is fixed to the first connecting body 510, such that the first connecting body 510 is suspended outside the arm body 100. In this way, a clearance is defined between the first connecting body 510 and the arm body 100 for accommodating a portion of the instrument driver 12. This prevents interference between the instrument driver 12 and the arm body 100 during installation, ensuring smooth movement of the first connecting component 400.

It should be noted that in the present disclosure, the first direction, the second direction, and the third direction are perpendicular to each other. For purposes of clarity and brief description, in the accompanying drawings, the first direction is defined as the X-axis direction, the second direction is defined as the Y-axis direction, and the third direction is defined as the Z-axis direction. Taking the arm body 100 as an example, the X-axis direction is the length direction of the arm body 100, the Y-axis direction is the thickness direction of the arm body 100, and the Z-axis direction is the width direction of the arm body 100.

As shown in FIG. 6 or FIG. 7, in some embodiments, the second connecting body 520 is configured as a block and passes through the through slot 120 in the second direction perpendicular to the first direction. The first connecting body 510 and the second connecting body 520 are mounted to the first connecting component 400 in the second direction from a side away from the first connecting component 400 toward the first connecting component 400. In this way, when assembling the second connecting body 520 to the first connecting component 400, it is only need to insert the second connecting body 520 into the first cavity 110 through the through slot 120 in the Y-axis direction, to complete the installation with the first connecting component 400 located in the first cavity 110, simplifying the operation.

In conjunction with the foregoing embodiments, as shown in FIG. 6, in some embodiments, the first connecting component 400 defines an insertion hole 420, and a portion of the second connecting body 520 is inserted into the insertion hole 420. In this way, after the portion of the second connecting body 520 passes through the through slot 120 in the Y-axis direction and then is inserted into the insertion hole 420, preliminary positioning and installation of the second connecting body 520 with the first connecting component 400 is achieved, facilitating subsequent fastening using a fastener 600.

As shown in FIG. 3 and FIG. 6, in some other embodiments, the first connecting component 400 defines a first connecting hole 430, the first connecting body 510 defines a first through hole 511, and the second connecting body 520 defines a second through hole 521. The fastener 600 has an end abutting against the first connecting body 510 and another end passing through the first through hole 511 and the second through hole 521 and fastened in the first connecting hole 430. By securing the fastener 600 in the first connecting hole 430, both the first connecting body 510 and the second connecting body 520 are fixed to the first connecting component 400, thereby reducing the number of fastening components as well as the fastening process, and improving the assembly efficiency of the instrument holder 13.

It should be noted that the fastener 600 may be configured in various forms, including but not limited to a screw, a rivet, a pin, and the like.

Further, in some embodiments, the first connecting hole 430 is an internally threaded hole, and the other end of the fastener 600 is provided with a screw rod 610 threadedly engaged in the internally threaded hole. The threaded fastening configuration allows the fastener 600 to be fixed to the first connecting component 400, thereby enabling simultaneous fixation of the first connecting body 510 and the second connecting body 520 to the first connecting component 400. This facilitates assembly while also allows for easy disassembly during maintenance.

Specifically, the fastener 600 includes at least one selected from a group consisting of a bolt and a screw.

In some embodiments, the first through hole 511 and the second through hole 521 are threaded through holes. The screw rod 610 of the fastener 600 is threadedly engaged in the first through hole 511 and the second through hole 521, thereby securing the first connecting body 510 and the second connecting body 520 to the fastener 600. Subsequently, the second connecting body 520 is inserted through the through slot 120, allowing the screw rod 610 of the fastener 600 to engage with the internally threaded hole of the first connecting component 400, thus fixing the first connecting body 510 and the second connecting body 520 to the first connecting component 400.

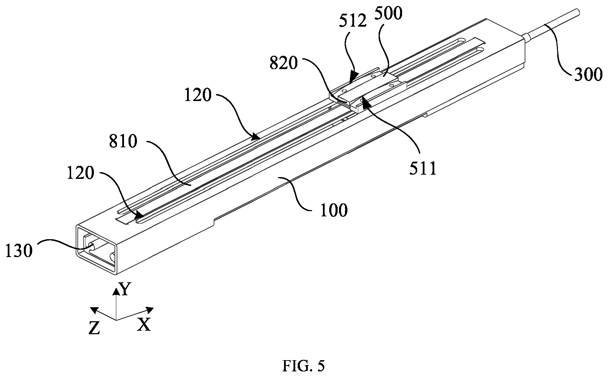

As shown in FIG. 5, in some embodiments, the first connecting component 400 defines at least two first connecting holes 430 spaced apart from each other in the first direction. The number of the first through holes 511, the number of the second connecting body 520, and the number of the fasteners 600 are each equal to the number of the first connecting holes 430; and the first through holes 511, the second through holes 521, and the fasteners 600 are arranged as one-to-one correspondence with the first connecting holes 430. In this way, at least two fastening points are defined between the first connecting body 510 and the second connecting body 520 with respect to the first connecting component 400, which helps to improve the connection strength therebetween.

In some embodiments, the second connecting body 520 is configured as a block and passes through the through slot 120 in the second direction perpendicular to the first direction, i.e., the thickness direction of the arm body 100. This allows the second connecting body 520 to pass through the through slot 120 without contacting the arm body 100, thereby preventing interference with the movement of the first connecting component 400.

In conjunction with the foregoing embodiments, as shown in FIG. 5 and FIG. 6, in some embodiments, the instrument holder 13 includes two second connecting bodies 520 spaced apart from each other in a third direction, where the first direction, the second direction, and the third direction are perpendicular to each other. The arm body 100 defines two through slots 120. The two second connecting bodies 520 respectively pass through the two through slots 120 to connect to the first connecting component 400 and the second connecting component 500. In this way, the two second connecting bodies 520 are spaced apart from each other in the third direction, and the two through slots 120 are likewise spaced apart from each other in the third direction, thereby defining a clearance that facilitates arrangement of other components, such as an encoder mover 820, on the first connecting body 510. Moreover, this effectively utilizes the space in the width direction of the arm body 100 to accommodate the two second connecting bodies 520, thereby enhancing the connection strength between the first connecting body 510 and the first connecting component 400.

In some embodiments, to enhance the strength of the arm body 100, particularly the strength of the side where the through slot 120 is located, the second connecting component 500 may include only one second connecting body 520, and accordingly, the arm body 100 defines only one through slot 120, as shown in FIG. 10.

In some embodiments, the first connecting body 510 is configured as a plate, which facilitates connection with the instrument driver 12, routing of cables, and the like. Additionally, the first connecting body 510 is coupled to the first connecting component 400 via the second connecting body 520 and four fasteners 600, such that a reliable connection between the first connecting body 510 and the first connecting component 400 is ensured.

In addition, when the configuration of the second connecting body 520 is in the shape of a block, the second connecting body 520 is in the shape of a rod or in the shape of a plate.

As shown in FIG. 3 and FIG. 6, in some embodiments, the second connector 520 is plate-shaped, and two plate-shaped second connecting bodies 520 are arranged in the third direction, and each second connecting body 520 defines two second through holes 521 in the first direction.

In some other embodiments, the second connecting body is rod-shaped, and four rod-shaped second connecting bodies 520 and four fasteners 600 are provided, with the four second connecting bodies 520 corresponding one-to-one with the four fasteners 600.

In some embodiments, referring to FIG. 3, the arm body 100 is configured as an integral structure. In some other embodiments, referring to FIG. 10, the arm body 100 is configured as a separate structure.

Referring to FIG. 10, the arm body 100 includes a first arm body 100A and a second arm body 100B. The first arm body 100A and the second arm body 100B collectively enclose to form the first cavity 110. The through slot 120 is defined in the second arm body 100B. Both the first arm body 100A and the second arm body 100B extend in the first direction X, that is, each of the first arm body 100A and the second arm body 100B has a length in the first direction X. Moreover, the projection of the first arm body 100A in the first direction X covers the projection of the second arm body 100B in the first direction X. In some embodiments, the length of the first arm body 100A may be regarded as the length of the first cavity 110, and the length of the second arm body 100B is less than or equal to the length of the first cavity 110. In other words, the first arm body 100A serves as the primary load-bearing part of the entire instrument holder 13 and defines a large opening. The second arm body 100B serves as a cover, which can enclose the opening of the first arm body 100A to a certain extent, such that the first arm body 100A and the second arm body 100B together define the first cavity 110. In the embodiments, the first mating surface is formed in the first cavity 110 of the arm body 100. Since the first arm body 100A has a separate structure and a relatively large opening, a tool can be easily inserted through the opening for operation.

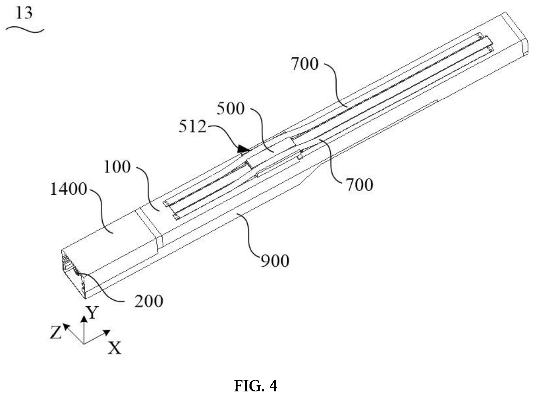

Based on any of the foregoing embodiments, referring to FIG. 3 and FIG. 4, in some embodiments, the instrument holder 13 further includes a shielding component 700 configured to cover the through slot 120 and at least a portion of the second connecting component 500. The shielding component 700 extends in the first direction, and two opposite ends of the shielding component 700 in the first direction are fixed to the arm body 100. The shielding component 700 covers the through slot 120, preventing solid debris such as dust from entering the first cavity 110 through the through slot 120 and contaminating the lead screw 300 and the first connecting component 400, thereby preventing a reduction in the service life of the lead screw 300 and the first connecting component 400. Additionally, the shielding component 700 covers at least a portion of the second connecting component 500, i.e., the shielding component 700 rests on the second connecting component 500 without interfering with the movement of the second connecting component 500, and can be easily lifted by the second connecting component 500.

Further, in some embodiments, when the second connecting component 500 moves between the first end and the second end of the through slot 120, the shielding component 700 dynamically covers the through slot 120. In this way, during the movement of the second connecting component 500, a portion of the shielding component 700 ahead of the second connecting component 500 in the movement direction of the second connecting component 500 is lifted, exposing the corresponding portion of the through slot 120, thus facilitating movement of the second connecting component 500. Meanwhile, a portion of the through slot 120 left behind as the second connecting component 500 moves away remains open, and the corresponding portion of the shielding component 700 conforms to the arm body 100 to promptly cover that portion of the through slot 120. That is, when the second connecting component 500 travels between the first end and the second end, the shielding component 700 can promptly open the portion of the through slot 120 ahead of the second connecting component 500 without interfering with the movement of the second connecting component 500, while promptly covering the portion of the through slot 120 behind the second connecting component 500. This effectively prevents foreign matter from entering the first cavity 110 through the through slot 120 and thereby enhancing the protective performance of the arm body 100.

Further, in some embodiments, one of the shielding component 700 and the arm body 100 is provided with a magnetic attraction member (not shown), and the other of the shielding component 700 and the arm body 100 is provided with a mating member (not shown) magnetically attracted and fixed to the magnetic attraction member. The magnetic attraction member and the mating member are magnetically coupled to each other, enabling the shielding component 700 to dynamically cover the through slot 120. Thus, during movement of the second connecting component 500, a portion of the shielding component 700 ahead of the second connecting component 500 is lifted by the second connecting component 500, exposing the corresponding portion of the through slot 120 to facilitate the movement of the second connecting component 500. Meanwhile, as the second connecting component 500 moves away, the portion of the through slot 120 left behind remains open, and the corresponding portion of the shielding component 700 promptly covers that portion of the through slot 120 by magnetic attraction between the magnetic attraction member and the mating member.

It should be noted that the magnetic attraction member includes magnetic attraction structure such as a magnet and a magnetic strip. The mating member includes a magnet, iron and other magnetic attraction structures. Referring back to FIG. 3 and FIG. 4, in some embodiments, the shielding component 700 includes a flexible shielding strip, and portions of the flexible shielding strip can be reset to conform to the arm body 100 to cover the through slot 120 after clearing the second connecting component 500, to cover the through slot 120. In this way, the flexibility of the flexible shielding strip allows it to be easily lifted by the second connecting component 500 to expose the through slot 120. Moreover, when the flexible shielding strip covers the portion of the through slot 120 behind the second connecting component 500, it can better conform to the arm body 100, preventing foreign matter from entering the first cavity 110 through the through slot 120 and thereby enhancing the protective performance of the arm body 100.

Alternatively, in some embodiments, the shielding component 700 is configured as a metal strip, a woven strip, a plastic strip, an elastic strip, a magnetic strip, or the like.

In any of the foregoing embodiments of the shielding component 700, referring back to FIG. 3 to FIG. 6, in some embodiments, the second connecting component 500 defines a first guiding groove 512 extending in the first direction. The shielding component 700 passes through the first guiding groove 512. The guidance provided by the first guiding groove 512 to the shielding component 700 enables smoother cooperation of the shielding component 700, the second connecting component 500, and the arm body 100, making the shielding component 700 less prone to derailment and thus ensuring high reliability of the shielding effect.

Alternatively, as shown in FIG. 5, in some embodiments, the second connecting component 500 partially passes through the through slot 120 in the second direction perpendicular to the first direction, and the bottom surface of the first guiding groove 512 has an arc-shaped projection on a plane defined by the first direction and the second direction. This facilitates an arc-shaped guiding cooperation between the shielding component 700 and the second connecting component 500, enabling smoother dynamic covering of the through slot 120 by the shielding component 700.

Still referring to FIG. 5, in some embodiments, in the first direction, the depth of the first guiding groove 512 relative to the through slot 120 gradually increases from an end of the first guiding groove 512 toward a middle of the first guiding groove 512. The bottom surface of the first guiding groove 512 smoothly transitions from an end to the other end. This facilitates smooth guiding engagement between the shielding component 700 and the second connecting component 500, allowing the shielding component 700 to dynamically cover the through slot 120 more smoothly.

Based on any of the foregoing embodiments, as shown in FIG. 3, in some embodiments, the instrument holder 13 further includes a position detection assembly 800 configured to detect a position state of the second connecting component 500 relative to the arm body 100. The position detection assembly 800 obtains the position status of the second connecting component 500 in real time to indirectly acquire position information of the instrument driver 12 in the first direction, enabling the control device 20 to control the actuator 200 to adjust the position of the instrument driver 12 to meet the requirements of surgical operation.

It should be noted that the position detection assembly 800 can be configured in various forms, including but not limited to a machine vision detection assembly, a linear encoder, and the like.

As shown in FIG. 5 and FIG. 6, in some embodiments, the position detection assembly 800 is configured as a linear encoder, which includes an encoder stator 810 fixed to the arm body 100 and an encoder mover 820 fixed to the second connecting component 500. The encoder stator 810 is configured to detect an instantaneous or continuous position, speed, or status of the encoder mover 820. In the embodiments, the encoder mover 820 moves along with the second connecting component 500 and cooperates with the encoder stator 810 to determine the position, speed, or status of the second connecting component 500.

In some embodiments, the encoder stator 810 at least includes a grating scale, and the encoder mover 820 at least includes a photosensitive element.

In some embodiments, the encoder stator 810 at least includes a magnetic scale, and the encoder mover 820 at least includes a magneto-sensitive element.

In some other embodiments, the position detection assembly 800 is a draw-wire encoder.

In some embodiments, the encoder stator 810 is offset from the through slot 120 to prevent interference to the motion of the second connecting component 500 and the first connecting component 400.

As shown in FIG. 5, in some embodiments, a portion of the second connecting component 500 passes through the through slot 120 in the second direction. The arm body 100 defines two through slots 120 spaced apart from each other in the third direction. The encoder stator 810 is located between the two through slots 120 and fixed to the arm body 100. The two through slots 120 are spaced apart from each other in the third direction, making full use of the width of the arm body 100 to create a clearance space. This facilitates placement of the encoder stator 810 in the clearance space and its connection to the arm body 100, avoiding interference to the motion of the second connecting component 500 and the first connecting component 400. The encoder mover 820 is fixed to the second connecting component 500. By integrating the encoder mover 820 on the second connecting component 500, the instrument holder 13 becomes more compact, and cable routing is facilitated via the second connecting component 500.

Based on any of the foregoing embodiments, as shown in FIG. 6, in some embodiments, the projection of the second mating surface 410 on a plane defined by the second direction and the third direction has a convex shape, and the projection of the first mating surface 130 on the plane defined by the second direction and the third direction has a concave shape. In this way, the engagement between the protrusion and the recess restricts rotation of the first connecting component 400 about the axis of the lead screw 300, offering ease of manufacture and low implementation cost.

As shown in FIG. 7, in some other embodiments, the projection of the second mating surface 410 on the plane defined by the second direction and the third direction has a concave shape, and the projection of the first mating surface 130 on the plane defined by the second direction and the third direction has a convex shape. In this way, the engagement between the protrusion and the recess restricts rotation of the first connecting component 400 about the axis of the lead screw 300, offering ease of manufacture and low implementation cost.

Based on any of the foregoing embodiments, as shown in FIG. 6 or FIG. 7, in some embodiments, the first connecting component 400 is provided with two second mating surfaces 410 that are symmetrically arranged in the third direction. The arm body 100 is correspondingly provided with two first mating surfaces 130 which are configured as one-to-one correspondence with the two second mating surfaces 410 and are respectively in constraining engagement with the two second mating surfaces 410. This ensures that the first connecting component 400 is subjected to uniform force in the width direction of the arm body 100. Additionally, through the one-to-one correspondence and constraining engagement between the two first mating surfaces 130 and the two second mating surfaces 410, the first connecting component 400 is more reliably restricted from rotating about the axis of the lead screw 300. This results in higher connection precision for the sliding connection between the first connecting component 400 and the arm body 100, which helps reduce the parallelism error between the lead screw 300 and the first direction, thereby improving the movement accuracy of the instrument driver 12 in the X-axis direction.

As shown in FIG. 6 or FIG. 7, in some embodiments, one of the first mating surface 130 and the second mating surface 410 defines a sliding groove 131, and the other of the first mating surface 130 and the second mating surface 410 is provided with a protrusion 411 that is engaged in the sliding groove 131 and slidable relative to the sliding groove 131. In this way, the cooperation between the sliding groove 131 and the protrusion 411 provides constraining engagement between the first mating surface 130 and the second mating surface 410, enabling the first connecting component 400 to be coupled to the arm body 100 and slidable relative to the arm body 100 in the length direction of the arm body 100.

In some embodiments, the first mating surface 130 defines the sliding groove 131, and the second mating surface 410 is provided with the protrusion 411 that is engaged in the sliding groove 131 and slidable relative to the sliding groove 131.

As shown in FIG. 6, in some embodiments, the protrusion 411 is at least partially cylindrical. In this way, the sliding groove 131 can receive the at least partially cylindrical protrusion 411 to prevent rotation of the first connecting component 400 about the axis of the lead screw 300.

In some other embodiments, the second mating surface 410 defines the sliding groove 131, and the first mating surface 130 is provided with the protrusion 411 that is engaged in the sliding groove 131 and slidable relative to the sliding groove 131.

As shown in FIG. 7, in some embodiments, the protrusion 411 includes a ridge 132 and an anti-disengagement member 133 fixed to an end of the ridge 132. The sliding groove 131 is configured to receive the anti-disengagement member 133 and is in slidable engagement with the anti-disengagement member 133, which enhances the engagement between the anti-disengagement member 133 and the sliding groove 131, improving the sliding connection precision between the first connecting component 400 and the arm body 100.

In some embodiments, a projection of the anti-disengagement member 133 onto the plane defined by the second direction and the third direction is a circular arc greater than a semicircle.

Based on any of the foregoing embodiments, as shown in FIG. 6 or FIG. 7, in some embodiments, the arm body 100 includes a profile member 140 and a guiding rail 150 fixed to the profile member 140. The first mating surface 130 is located on the guiding rail 150. The arm body 100 is formed by assembling the profile member 140 and the guiding rail 150, which can reduce the manufacturing difficulty and cost of the arm body 100.

As described above, the instrument holder 13 is disposed at the distal end of the manipulator 11. In some embodiments, to facilitate interaction and reduce the number of cables, the instrument holder 13 further includes a circuit board assembly 1100, which is coupled to the actuator 200, the position detection assembly 800, and the instrument driver 12.

Referring to FIG. 3, FIG. 4, and FIG. 6, in some embodiments, the instrument holder 13 further includes a housing 900 fixed to the profile member 140. A second cavity 910 is defined between the housing 900 and the profile member 140, facilitating assembly. The circuit board assembly 1100 is disposed within the second cavity 910 and is electrically coupled to the actuator 200. The second cavity 910 protects the circuit board assembly 1100 to prevent damage to the circuit board assembly 1100 from affecting the operation of the actuator 200, and thus enhancing the electrical reliability of the instrument holder 13.

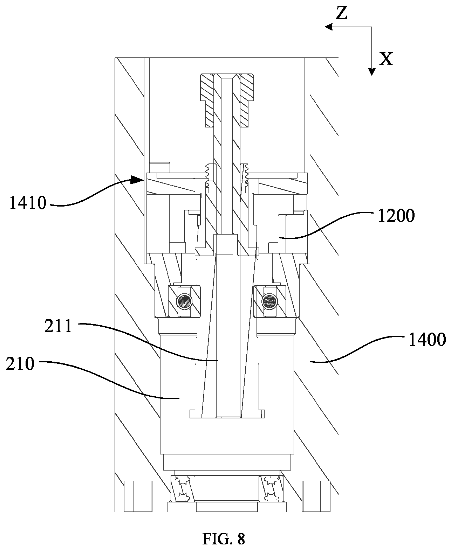

Based on any of the foregoing embodiments, as shown in FIG. 3 and FIG. 8, in some embodiments, the actuator 200 includes a power source 210 fixed to the arm body 100. The power source 210 is drivingly coupled to the lead screw 300. At least a portion of the lead screw 300 is disposed within the first cavity 110 and is threadedly engaged with the first connecting component 400. Thus, the first connecting component 400 is threadedly engaged with the lead screw 300 and is slidable relative to the arm body 100. When the power source 210 drives the lead screw 300 to rotate, the first connecting component 400 is driven to reciprocate in the axial direction of the lead screw 300, thereby moving the instrument driver 12 in the length direction of the arm body 100. Moreover, due to the self-locking property of the threaded engagement, the instrument driver 12 can be stopped at any desired position.

Based on the foregoing embodiments, the power source 210 is protected by the housing 900, which provides good protection while not affecting the electrical connection between the power source 210 and the circuit board assembly 1100. The actuator 200 is fixed to the profile member 140, and the circuit board assembly 1100 is fixed to the profile member 140, with the two being electrically coupled via a cable. Finally, by fixing the housing 900 to the profile member 140, the actuator 200 and the arm body 100 are integrated with good electrical protection.

As shown in FIG. 8, in some embodiments, the power source 210 includes an output shaft 211 drivingly coupled to the lead screw 300, and the instrument holder 13 further includes a second detection assembly 1200 configured to detect a rotation angle of the output shaft 211, so as to obtain power output information of the power source 210, thereby facilitating control over the power source 210.

It should be noted that the second detection assembly 1200 may be configured in various forms, including but not limited to a rotary encoder, an angular displacement sensor, and the like.

As shown in FIG. 3, FIG. 4, and FIG. 8, in some embodiments, the instrument holder 13 further includes an end housing 1400 fixed to an end of the arm body 100. The end housing 1400 defines a driving cavity 1410, and at least a portion of the actuator 200 is disposed within the driving cavity 1410. Thus, at least a portion of the actuator 200 is mounted on the arm body 100 via the end housing 1400, and is protected in the driving cavity 1410, thereby improving the protective performance of the instrument holder 13.

In some embodiments, the end housing 1400 is mounted to the arm body 100 in the first direction from a side away from the arm body 100 toward the arm body 100, thereby improving assembly efficiency.

In the embodiments, the actuator 200 includes the power source 210. The power source 210 is mounted on the end housing 1400, and then the end housing 1400 is mounted to the arm body 100 in the first direction from the side away from the arm body 100 toward the arm body 100, thereby facilitating assembly.

In some embodiments, the end housing 1400 is fixed to the housing 900. This not only enhances the protective effect on the actuator 200 but also improves the connection strength between the actuator 200 and the arm body 100.

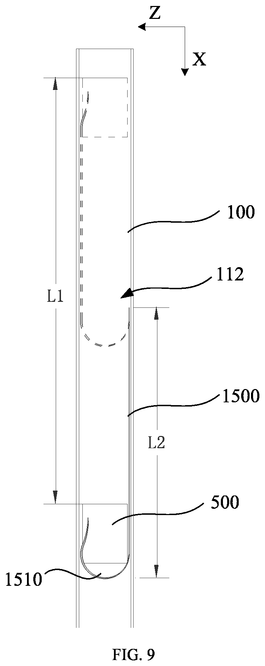

Due to the need to supply power and control the instrument driver 12, a cable is typically provided on the instrument holder 13 for electrical connection with the instrument driver 12. Since the instrument driver 12 moves in the length direction of the arm body 100, at least a portion of the cable moves with the instrument driver 12 to ensure reliable electrical connection. Based on any of the foregoing embodiments, as shown in FIG. 6 and FIG. 9, in some embodiments, the first cavity 110 includes a transmission area 111 and a cable routing area 112. The lead screw 300 is located in the transmission area 111, and the first connecting component 400 moves within the transmission area 111. The cable routing area 112 is configured for routing the cable. The transmission area 111 and the cable routing area 112 are arranged in the second direction perpendicular to the first direction. This allows for a more organized interior of the arm body 100 and reduces interference between the first connecting component 400, the lead screw 300, and the cable. The cable routing area 112 is closer to the through slot 120 than the transmission area 111, thereby allowing the cable within the cable routing area 112 to be positioned away from the first connecting component 400, minimizing interference with the first connecting component 400 and the lead screw 300.

As shown in FIG. 9, in some embodiments, the instrument holder 13 further includes a cable carrier 1500. The cable carrier 1500 includes an end fixed to the second connecting component 500 and another end fixed to the arm body 100. In this way, the cable carrier 1500 moves with the second connecting component 500, and a movable portion of the cable carrier 1500 is confined within the cable routing area 112. In some embodiments, the arm body 100 further defines two second guiding grooves 101 facing the cable routing area 112, each second guiding groove 101 having a bottom surface parallel to and facing the other. At least a portion of the cable carrier 1500 is in dynamic guiding engagement with one of the two second guide grooves 101, and at least a portion of the cable carrier 1500 is in dynamic guiding engagement with the other of the two second guide grooves 101. Thus, the guiding engagement between the cable carrier 1500 and the second guiding grooves 101 facilitates movement of the cable carrier 1500 in the length direction of the second guiding grooves 101, allowing the movable portion of the cable to be arranged on the cable carrier 1500, thereby preventing the cable from interfering with the lead screw 300.

It should be noted that the cable carrier 1500 may be configured in various forms, including but not limited to a drag chain, a metal strip, or the like, as long as it is capable of accommodating the cable and guiding the cable to move in a predetermined path. For example, the cable is attached to an outer surface of the cable carrier 1500. Alternatively, the cable is clamped inside the cable carrier 1500.

In some embodiments, the other end of the cable carrier 1500 is coupled to the arm body 100 and disposed close to the other second guiding groove 101, such that a portion of the cable carrier 1500 defines a resilient bent portion 1510. The resilient bent portion 1510 abuts against the bottom walls of the two second guiding grooves 101. As such, the resilient bent portion 1510 enables the cable carrier 1500 to be snapped into the second guiding grooves 101 by its own elastic force, allowing the cable carrier 1500 to move in the extending direction of the second guiding grooves 101 without disengaging therefrom. This effectively prevents the cable carrier 1500 and the cable from scratching the lead screw 300 and affecting the transmission reliability of the lead screw 300.

As shown in FIG. 9, in some embodiments, a movable length of the cable carrier 1500 is greater than half of a total stroke of the second connecting component 500, such that the cable carrier 1500 does not interfere with the movement of the second connecting component 500, thereby avoiding a reduction in the stroke of the second connecting component 500.

As shown in FIG. 9, the movable length of the cable carrier 1500 is denoted as L1, and the total stroke of the second connecting component 500 is denoted as L2, satisfying 0.5*L2 ≤ L1.

It should be noted that the movable length of the cable carrier 1500 refers to the length of the portion of the cable carrier 1500 that is movable within the cable routing area 112, excluding the fixed portion of the cable carrier 1500.

The above are only some embodiments of the present disclosure, and neither the words nor the drawings can limit the protection scope of the present disclosure. Any equivalent structural transformation made by using the contents of the specification and the drawings of the present disclosure under the overall concept of the present disclosure, or directly/indirectly applied in other related technical fields are included in the protection scope of the present disclosure.

## Claims

1. An instrument holder, comprising:
an arm body, the arm body defining a first cavity extending between two ends of the arm body in a first direction, the arm body further defining a through slot for communicating the first cavity with an exterior of the arm body, the through slot extending in the first direction and comprising a first end and a second end oppositely disposed in the first direction, and the arm body comprising a first mating surface disposed within the first cavity;
an actuator fixed to an end of the arm body;
a lead screw, at least a portion of the lead screw extending within the first cavity, the lead screw being coupled to the actuator and configured to be driven by the actuator to rotate about an axis parallel to the first direction;
a first connecting component located within the first cavity, the first connecting component being threadedly engaged with the lead screw and configured to be driven by the lead screw to reciprocate in an axial direction of the lead screw, and a surface of the first connecting component defining a second mating surface; and
a second connecting component configured for mounting an instrument driver, the second connecting component being partly located outside the arm body and passing through the through slot to be fixed to the first connecting component; wherein,
the first connecting component and the second connecting component are reciprocatable between the first end and the second end of the through slot;
the first mating surface mates with the second mating surface to prevent the first connecting component from rotating about the axis of the lead screw; and
the second connecting component is free of contact with the arm body.

2. The instrument holder of claim 1, wherein, the second connecting component comprises:
a first connecting body configured for mounting the instrument driver, at least a portion of the first connecting body being located outside the arm body; and
a second connecting body, the second connecting body comprising an end located within the first cavity and fixed to the first connecting component, and the other end fixed to the first connecting body.

3. The instrument holder of claim 2, wherein,
the first connecting body and the second connecting body are integrally formed;
or
the first connecting body and the second connecting body are configured as separate structures.

4. The instrument holder of claim 2 or claim 3, wherein,
the second connecting body passes through the through slot, and the other end of the second connecting body is located outside the arm body and is fixed to the first connecting body, to allow the first connecting body to be suspended outside the arm body.

5. The instrument holder of any one of claims 2 to 4, wherein,
the second connecting body passes through the through slot in a second direction perpendicular to the first direction, and the first connecting body and the second connecting body are mounted to the first connecting component in the second direction from far to near.

6. The instrument holder of any one of claims 2 to 5, wherein,
the first connecting component defines an insertion hole, and a portion of the second connecting body is inserted into the insertion hole.

7. The instrument holder of any one of claims 2 to 5, wherein,
the first connecting component defines a first connecting hole, the first connecting body defines a first through hole, and the second connecting body defines a second through hole; and
the instrument holder further comprises:
a fastener, the fastener comprising an end abutting against the first connecting body and the other end passing through the first through hole and the second through hole and fastened in the first connecting hole.

8. The instrument holder of claim 7, wherein,
the first connecting hole is an internally threaded hole, and the other end of the fastener is provided with a screw rod threadedly engaged in the internally threaded hole.

9. The instrument holder of claim 7 or claim 8, wherein,
the first connecting component comprises at least two first connecting holes spaced apart from each other in the first direction; and
the first through hole is one of at least two first through holes, the second connecting body is one of at least two second connecting bodies, and the fastener is one of at least two fasteners; wherein,
a quantity of the at least two first through holes, a quantity of the at least two second connecting bodies, and a quantity of the at least two fasteners are each equal to a quantity of the at least two first connecting holes; and
the at least two first through holes, the at least two second connecting bodies, and the at least two fasteners are arranged as one-to-one correspondence with the at least two first connecting holes, respectively.

10. The instrument holder of claim 1 or claim 2, wherein,
the two second connecting bodies are spaced apart from each other in a third direction, and the two second connecting bodies respectively pass through the two through slots in a second direction to connect with the first connecting component and the second connecting component; wherein the first direction, the second direction, and the third direction are perpendicular to one another.

11. The instrument holder of claim 1 or claim 2, wherein,
the through slot is a single through slot, and the second connecting body is a single second connecting body.

12. The instrument holder of any one of claims 1 to 11, wherein,
the arm body is configured as an integral structure;
or
the arm body is configured as a separate structure.

13. The instrument holder of any one of claims 1 to 11, wherein,
the arm body comprises:
a first arm body extending in the first direction; and
a second arm body extending in the first direction; wherein,
a projection of the first arm body in the first direction covers a projection of the second arm body in the first direction, the first arm body and the second arm body enclose to define the first cavity, and the through slot is defined in the second arm body.

14. The instrument holder of any one of claims 1 to 13, further comprising:
a shielding component covering the through slot and at least a portion of the second connecting component, the shielding component extending in the first direction, and two opposite ends of the shielding component in the first direction being fixed to the arm body.

15. The instrument holder of claim 14, wherein,
the shielding component dynamically covers the through slot when the second connecting component moves between the first end and the second end of the through slot.

16. The instrument holder of claim 14 or claim 15, wherein,
one of the shielding component and the arm body is provided with a magnetic attraction member, and the other one of the shielding component and the arm body is provided with a mating member, wherein the magnetic attraction member and the mating member are magnetically fixed to each other, to allow the shielding component to dynamically cover the through slot.

17. The instrument holder of any one of claims 14 to 16, wherein,
the shielding component comprises a flexible shielding strip, wherein a portion of the flexible shielding strip is capable of conforming to the arm body after clearing the second connecting component, to cover the through slot.

18. The instrument holder of any one of claims 14 to 17, wherein,
the shielding component is configured as one selected from a group consisting of a metal strip, a woven strip, a plastic strip, an elastic strip, and a magnetic strip.

19. The instrument holder of any one of claims 14 to 18, wherein,
the second connecting component defines a first guiding groove extending in the first direction, and the shielding component passes through the first guiding groove.

20. The instrument holder of claim 19, wherein,
a portion of the second connecting component passes through the through slot in a second direction perpendicular to the first direction, and a projection of a bottom surface of the first guiding groove on a plane defined by the first direction and the second direction is arc-shaped; and/or,
a depth of the first guiding groove gradually increases from an end of the first guiding groove to a middle of the first guiding groove in the first direction, and a bottom surface of the first guiding groove transitions smoothly from an end to the other end.

21. The instrument holder of any one of claims 1 to 20, further comprising:
a position detection assembly configured to detect a position of the second connecting component relative to the arm body.

22. The instrument holder of claim 21, wherein,
the position detection assembly is a linear encoder, and the linear encoder comprises:
an encoder mover fixed to the second connecting component; and
an encoder stator fixed to the arm body, the encoder stator being configured to detect an instantaneous position, an instantaneous speed, or an instantaneous state of the encoder mover, or to detect a continuous position, a continuous speed, or a continuous state of the encoder mover.

23. The instrument holder of claim 22, wherein,
the encoder stator at least comprises a grating scale, and the encoder mover at least comprises a photosensitive element; or,
the encoder stator at least comprises a magnetic scale, and the encoder mover at least comprises a magneto-sensitive element.

24. The instrument holder of claim 22 or claim 23, wherein,
the encoder stator is offset from the through slot.

25. The instrument holder of any one of claims 22 to 24, wherein,
a portion of the second connecting component passes through the through slot in a second direction;
the arm body defines two through slots, and the two through slots are spaced apart from each other in a third direction; and
the encoder stator is located between the two through slots and fixed to the arm body, and the encoder mover is fixed to the second connecting component;
wherein the first direction, the second direction, and the third direction are perpendicular to one another.

26. The instrument holder of any one of claims 1 to 25, wherein,
a portion of the second connecting component passes through the through slot in a second direction; and
a projection of the second mating surface on a plane defined by the second direction and a third direction comprises a convex shape, and a projection of the first mating surface on the plane defined by the second direction and the third direction comprises a concave shape; and/or, a projection of the second mating surface on the plane defined by the second direction and the third direction comprises a concave shape, and a projection of the first mating surface on the plane defined by the second direction and the third direction comprises a convex shape;
wherein the first direction, the second direction, and the third direction are perpendicular to one another.

27. The instrument holder of claim 26, wherein,
the first connecting component comprises two second mating surfaces, and the two second mating surfaces are symmetrically arranged in the third direction; and
the arm body comprises two first mating surfaces; wherein,
the two first mating surfaces are configured as one-to-one correspondence with the two second mating surfaces, and the two first mating surfaces are respectively in constraining engagement with the two second mating surfaces.

28. The instrument holder of any one of claims 1 to 25, wherein,
one of the first mating surface and the second mating surface defines a sliding groove, and the other one of the first mating surface and the second mating surface is provided with a protrusion that is engaged in the sliding groove and slidable relative to the sliding groove.

29. The instrument holder of claim 28, wherein,
the protrusion is at least partially cylindrical;
or
the protrusion comprises a ridge and an anti-disengagement portion fixed at an end of the ridge, wherein the sliding groove is configured to receive the anti-disengagement portion, and the anti-disengagement portion is slidable relative to the sliding groove.

30. The instrument holder of claim 29, wherein,
a portion of the second connecting component passes through the through slot in a second direction; and
a projection of the anti-disengagement portion on a plane defined by the second direction and a third direction is a circular arc that is greater than a semicircle;
wherein the first direction, the second direction, and the third direction are perpendicular to one another.

31. The instrument holder of any one of claims 1 to 30, wherein,
the arm body comprises a profiled member and a guiding rail fixed to the profiled member;
wherein the first mating surface is located on the guiding rail.

32. The instrument holder of claim 31, further comprising:
a housing fixed to the profiled member, a second cavity being defined between the housing and the profiled member; and
a circuit board assembly disposed within the second cavity, the circuit board assembly being electrically coupled with the actuator.

33. The instrument holder of any one of claims 1 to 32, wherein,
the actuator comprises:
a power source fixed relative to the arm body, the power source being drivingly coupled to the lead screw;
wherein at least a portion of the lead screw is disposed within the first cavity and is threadedly engaged with the first connecting component.

34. The instrument holder of claim 33, wherein,
the power source comprises an output shaft drivingly coupled to the lead screw; and
the instrument holder further comprises a second detection assembly configured to detect a rotation angle of the output shaft.

35. The instrument holder of any one of claims 1 to 34, wherein,
a portion of the second connecting component passes through the through slot in a second direction perpendicular to the first direction; and
the first cavity comprises a transmission area and a cable routing area; wherein,
the lead screw is located in the transmission area, and the first connecting component moves within the transmission area, the cable routing area is configured for arranging a cable, and the transmission area and the cable routing area are arranged in the second direction; and
the cable routing area is closer to the through slot than the transmission area.

36. The instrument holder of claim 35, wherein,
the arm body defines two second guiding grooves facing the cable routing area, bottom surfaces of the two second guiding grooves are parallel and facing each other;
the instrument holder further comprises a cable carrier; wherein,
the cable carrier comprises an end fixed to the second connecting component and another end fixed to the arm body; and
at least a portion of the cable carrier is in dynamically guiding engagement with one of the two second guiding grooves, and at least another portion of the cable carrier is in dynamically guiding engagement with the other of the two second guiding grooves.

37. The instrument holder of claim 36, wherein,
the other end of the cable carrier is disposed adjacent to the other of the two second guiding grooves, to allow a portion of the cable carrier forms a resilient bent portion, wherein the resilient bent portion abuts against bottom walls of the two second guide grooves;
and/or
a movable length of the cable carrier is greater than half of a total stroke of the second connecting component.

38. The instrument holder of any one of claims 1 to 37, further comprising:
an end housing fixed to an end of the arm body, the end housing defining a driving cavity; wherein,
at least a portion of the actuator is located within the driving cavity.

39. The instrument holder of claim 38, wherein,
the end housing is mounted to arm body in the first direction from far to near.

40. A multi-axis motion device, comprising:
a manipulator;
an instrument driver; and
the instrument holder of any one of claims 1 to 39; wherein,
the instrument holder is disposed at a distal end of the manipulator, and the instrument driver is coupled to the second connecting component of the instrument holder.

41. A surgical robot, comprising the multi-axis motion device of claim 40.
